# EUROPÄISCHE PATENTSCHRIFT

(11) **EP 0 741 587 B1**
(45) Veröffentlichungstag und Bekanntmachung des Hinweises auf die Patenterteilung: **17.03.1999**
(21) Anmeldenummer: 95906350.4
(22) Anmeldetag: 26.01.1995
(51) Int. Cl.: A61M 5/142

(54) **IMPLANTIERBARES DOSIERSYSTEM**
IMPLANTABLE DOSAGING SYSTEM
SYSTEME DE DOSAGE POUVANT ETRE IMPLANTE

(30) Priorität: 27.01.1994 DE 4402380
(43) Veröffentlichungstag der Anmeldung: 13.11.1996
(73) Patentinhaber: Zenner, Hans Peter, Prof. Dr. med., 72070 Tübingen (DE); Lehner, Rolf, 73734 Esslingen (DE)
(72) Erfinder: Zenner, Hans Peter, Prof. Dr. med., 72070 Tübingen (DE); Lehner, Rolf, 73734 Esslingen (DE)
(74) Vertreter: Patentanwälte Ruff, Beier, Schöndorf und Mütschele
(86) Internationale Anmeldenummer: EP9500280
(87) Internationale Veröffentlichungsnummer: WO9520409

(56) Entgegenhaltungen:
- EP-A- 0 259 668
- EP-A- 0 335 671
- EP-A- 0 450 186
- DE-A- 3 600 217
- US-A- 4 013 074
- US-A- 4 443 218

## Beschreibung

Die Erfindung betrifft ein implantierbares Dosiersystem für Medikamente, Wirkstoffe oder dgl., die in Form von insbesondere gelösten oder suspendierten Fluiden appliziert werden, nach dem Oberbegriff von Anspruch 1, wie es beispielsweise aus der EP-A-450186 bekannt ist.

Aufgrund der Tatsache, daß eine Vielzahl von Medikamenten an verschiedenen Stellen des Körpers (ubiquitär) wirkt, ist es wünschenswert, diese Medikamente möglichst zielgerichtet an die Stelle oder die Stellen des Organismus zu bringen, an denen sie ihre Wirkung entfalten sollen. Bei systemischer Verabreichung ubiquitär wirkender Medikamente können teilweise beachtliche Nebenwirkungen beobachtet werden. Eine orale Verabreichung bestimmter Wirkstoffe ist nicht immer möglich, da diese im Verdauungstrakt teilweise abgebaut werden. So besteht also in mehrerer Hinsicht ein Bedürfnis, Medikamente oder Wirkstoffe direkt (intracorporal lokal) zu applizieren.

Die lokale intracorporale Applikation ist jedoch bei einer Vielzahl von Erkrankungen oder Beschwerden kompliziert. Wird das Medikament beispielsweise lokal injiziert, so muß diese Injektion bei nahezu jeder Anwendung durch geschulte Therapeuten gesetzt werden. Dies bedeutet im Normalfall, daß für jede Applikation ein Arzt aufgesucht werden muß. Außerdem sind die meisten Regionen des Organismus für eine entsprechende Anwendung schwer zugänglich, was insbesondere für den Schädelbereich des Menschen gilt. So muß beispielsweise bei bestimmten Behandlungen des Mittel- oder Innenohres ein Medikament durch das Trommelfell in die Paukenhöhle appliziert werden. Dies ist nicht nur medizinisch aufwendig, sondern für den Patienten auch belastend.

Aus dem Stand der Technik sind bereits eine Reihe von implantierbaren Dosiersystemen bekannt. Nach der DE 3639980 C1 besteht ein Dosiersystem aus einer implantierbaren Kapsel, in der ein Medikamenten-Sammelbehälter und eine Pumpeinrichtung untergebracht ist. Zum Wiederauffüllen des Sammelbehälters ist eine implantierbare Katheteranordnung mit einem Zuspritzport vorgesehen. Das Dosiersystem ist nur für Grobdosierungen mit Einzeldosen von beispielsweise 2 ml geeignet.

Die EP 0 174 757 A1 beschreibt ein Dosiersystem, bei dem die einzelnen Bauelemente wie Vorratsbehälter, Nachfüllstelle, Pumpe und Katheter über Verbindungsleitungen miteinander verbunden sind. Die Bauelemente selbst sind aus Elastomermaterial hergestellt. Derartige Dosiersysteme sind aufgrund des Aufbaus aus einer Vielzahl verschiedener Teile schwierig zu implantieren.

Die EP 0 335 671 A1 offenbart ein Dosiersystem, bei dem die wesentlichen Bauteile zu einer einzigen Baueinheit zusammengefaßt sind. Die Größe des Vorratsraums, in dem sich das zu applizierende Medikament befindet, ist jedoch im Verhältnis zum Gesamtvolumen des Dosiersystems gering. Außerdem besitzt die vorgesehene Pumpeinrichtung einen komplizierten Aufbau.

Die Funktionsfähigkeit des Systems insgesamt beruht ausschließlich auf der Elastizität seiner Bauteile.

Die drei europäischen Offenlegungsschriften EP 0 342 945 A2, EP 0 342 946 A2 und EP O 342 947 A2 zeigen ein ganz ähnliches Dosiersystem wie in der bereits beschriebenen EP 0 335 671 A1. Auch hier ist die verwendete Pumpeinrichtung sehr aufwendig konstruiert und die Menge des im Vorratsraum aufnehmbaren Medikaments im Verhältnis zum Gesamtvolumen des insgesamt beanspruchten Raumes gering. Auch hier beruht die Funktionsfähigkeit des Dosiersystems auf der Verformbarkeit des die Pumpeinrichtung enthaltenden Gehäuses und der Pumpeinrichtung selbst.

In der EP 0 450 186 A1 ist ein Dosiersystem beschrieben, das einen separaten Medikamentenspeicher und eine sogenannte Betätigungseinheit aufweist. Medikamentenspeicher und Betätigungseinheit sind über eine Verbindungsleitung miteinander verbunden. Die Betätigungseinheit umfasst ein sogenanntes Einspritzport, eine Pumpeinrichtung und ein Ventil. Dabei ist die Betätigungseinheit allerdings so konstruiert, daß zur Applikation des Medikaments immer eine in zwei Stufen bewirkte gleichzeitige Betätigung erforderlich ist. So muß zunächst durch einen Finger das Ventil manuell geöffnet und in der geöffneten Stellung gehalten werden. Dann muß durch einen zweiten Finger zusätzlich die Pumpeinrichtung betätigt werden. Erst dann kann das Medikament ausfließen.

Die Erfindung stellt sich die Aufgabe, die aus dem Stand der Technik bekannten Nachteile zu vermeiden. So soll insbesondere der Aufbau des Dosiersystems möglichst einfach und dementsprechend wenig anfällig für Störungen sein. Weiter soll erreicht werden, daß möglichst geringe, exakt definierte Wirkstoffmengen einfach appliziert und das System ohne großen Aufwand viele Male wiederbefüllt werden kann. Gleichzeitig soll ein im Verhältnis zu den Gesamtabmessungen des Dosiersystems möglichst großer Vorratsraum geschaffen werden, um möglichst viele Einzelapplikationen durchführen zu können, bevor der Vorratsraum wiederbefüllt werden muß. Schließlich soll ein erfindungsgemäßes Dosiersystem für eine mindestens teilweise Implantation in Knochen oder Knochenhöhlen geeignet sein.

Diese Aufgabe wird gelöst durch ein Dosiersystem mit den Merkmalen des Anspruchs 1. Bevorzugte Ausführungsformen der Erfindung sind in den Unteransprüchen beschrieben.

Das erfindungsgemäße Dosiersystem weist die folgenden Bestandteile auf:
- einen separaten Medikamentenspeicher,
- ein Gehäuse mit
   einer innerhalb des Gehäuses angeordneten Pumpe,
   die mit Hilfe eines am Gehäuse befindlichen Betätigungselements transkutan betätigbar ist,
   einer Austrittsöffnung für das zu applizierende Medikament,
   einem am Auslaß der Pumpe angeordneten Ventil,
   einer sich selbsttätig verschließenden Öffnung,
   insbesondere zur Wiederbefüllung des Medikamentenspeichers,
- eine Verbindungsleitung zwischen Gehäuse und Medikamentenspeicher,
- und ggf. eine von der Austrittsöffnung zum Applikationsort führende Dosierleitung.

Dabei handelt es sich bei der Pumpe um eine Tauchkolbenpumpe. Das Ventil ist als fluiddruckabhängig öffnendes Schlauchventil ausgebildet und die Austrittsöffnung kann durch Betätigung des Betätigungselements der Pumpe über das Schlauchventil reversibel mit Fluid beaufschlagt werden.

Durch das von der Erfindung vorgeschlagene Dosiersystem wird eine Vielzahl von Vorteilen erreicht. Es ergibt sich ein einfacher, kompakter Aufbau aus relativ wenigen Bauteilen, die selbst wiederum einfach aufgebaut und konstruiert sein können. Dies führt nicht nur zu vergleichsweise geringen Kosten bei der Herstellung des Systems, sondern gewährleistet auch eine störungsfreie Funktion des Dosiersystems bei seinem Einsatz. Auch äußere Einflüsse wie beispielsweise Erschütterungen, Temperatur-, Lage- und Druckwechsel können die Funktion des Systems nicht negativ beeinflussen. Das erfindungsgemäße Dosiersystem ist transkutan, insbesondere durch den Finger des Patienten betätigbar. Für die Einzelapplikation ist kein Fachpersonal nötig. Weiter ist kein Energiespeicher, wie beispielsweise ein Akku oder eine Knopfzelle, oder ein Zusatzsystem wie eine Bedienungseinheit oder eine Fernsteuerung erforderlich. Gleichzeitig kann das System transkutan im implantierten Zustand gereinigt, desinfiziert und wiederbefüllt werden. Das Nutzvolumen des Systems für das zu applizierende Medikament ist im Verhältnis zum insgesamt vom System in Anspruch genommenen Raum vergleichsweise groß. Dies bedeutet, daß viele Einzelapplikationen vorgenommen werden können, bevor der Medikamentenspeicher nachgefüllt werden muß. Dieser Vorteil zeigt sich auch daran, daß ein getrennter (ausgelagerter) Medikamentenspeicher vorgesehen ist, der an einer anderen Stelle im Organismus implantiert wird als das die Pumpe aufweisende Bauteil. Das Volumen des Medikamentenspeichers kann groß gewählt werden. Durch die Einbringung des Dosiersystems direkt am oder in der Nähe des Ortes, an dem das Medikament oder der Wirkstoff wirken soll, werden die bereits geschilderten Nebenwirkungen ubiquitär wirkender Medikamente erheblich reduziert.

Durch die beim erfindungsgemäßen Dosiersystem ggf. vorgesehene Dosierleitung zwischen Austrittsöffnung der Pumpe und Applikationsort des Medikaments wird erreicht, daß die Applikation exakt an einem vorgewählten Ort des Organismus vorgenommen werden kann.

Die wesentlichen Bauteile des Dosiersystems, insbesondere die mit dem Organismus in Kontakt kommenden Bauteile wie das Gehäuse, können aus jedem beliebigen biokompatiblen Material aufgebaut sein. Dementsprechend ist auch ein Einsatz des Systems an jeder beliebigen Stelle des Organismus möglich. Bevorzugt ist ein Dosiersystem, bei dem das die Pumpe enthaltende Gehäuse aus einem formstabilen, im wesentlichen starren Material ausreichender Festigkeit aufgebaut ist. Dabei kann es sich um einen entsprechenden Kunststoff, insbesondere Polyurethan (PUR) oder Polysulfon (PSU) und/oder ein entsprechendes Metall, insbesondere (Rein)Titan handeln. Titan besitzt im Vergleich zu Kunststoff eine hohe Festigkeit und erlaubt den Einsatz dünnwandiger Bauteile. Außerdem unterstützt seine, ggf. durch geeignete Oberflächenbehandlung verstärkte, Affinität zum Knochengewebe das Einwachsen des Gehäuses in ein knöchernes Implantatlager. Durch Verwendung eines starren Gehäuses wird erreicht, daß ggf. mit Ausnahme von Teilen des Betätigungselements und eines elastischen separaten Medikamentenspeichers keine elastischen, deformierbaren Teile vorhanden sind. Dies macht diese Ausführungsformen besonders für einen Einsatz im Bereich von Knochen, insbesondere der Schädelknochen des Menschen geeignet. Ein Dosiersystem mit starrem (Pumpen-)Gehäuse kann besonders gut in eine entsprechende Ausnehmung des Knochens eingebracht, bei Bedarf dort befestigt werden.

Der separate Medikamentenspeicher besteht vorzugsweise aus einem elastischen und damit verformbaren (biokompatiblen) Material, insbesondere aus einem elastischen Kunststoffmaterial. Die erforderliche Elastizität kann beispielsweise auch durch einen Metallfaltenbalg erreicht werden. Ein solcher elastischer Speicher (Reservoir) kann sich ggf. in einem weiteren, vorzugsweise starren, Gehäuse aus den bereits genannten biokompatiblen Materialien befinden. Separate Medikamentenspeicher sind üblicherweise ebenfalls zur Implantation vorgesehen, insbesondere unter die Haut durch Fixieren (Annähen) an die Muskelfaszie, vorzugsweise im Bereich der Achselhöhle, des Halses oder des Hinterhauptes.

Für ggf. vorhandene weitere elastische Bauteile (Katheter, Membrane) sind Polymere wie Silikone, Polyurethane (PUR) und Teflon® (PTFE) verwendbar. Federelastische Teile, wie z.B. Rückstellfedern, können aus Titan oder auch hochlegierten implantierbaren Edelstählen bestehen. Innenliegende Systembauteile, wie Pumpenteile, z.B. Ventile, können ebenfalls aus Titan aufgebaut sein.

Das Gehäuse kann aus einem Teil (einstückig) gebildet sein. In Weiterbildung besteht das Gehäuse aber vorzugsweise aus einem Gehäusebasisteil und einem auf dieses Basisteil aufgesetzten Gehäusedeckel. Die Verbindung des Basisteils und des Deckels kann in jeder beliebigen Weise, beispielsweise durch Verschrauben, Verkleben, Verschweißen, Einrasten des Deckels usw. vorgenommen werden.

Wie bereits beschrieben, kann der Einsatz des erfindungsgemäßen Dosiersystems überall im Organismus, vorzugsweise im Bereich von Knochen und Knochenhöhlen, vorgesehen sein. Wie bereits angedeutet, kann dabei das gesamte System an der ausgewählten Stelle implantiert werden oder auch nur ein Teil des Gesamtsystems, wie vorzugsweise das Gehäuse mit Betätigungselement, Pumpe, ggf. Dosierleitung und Befüllöffnung. Der separate, insbesondere elastische, Medikamentenspeicher mit oder ohne Befüllöffnung ist dann beispielsweise an anderer Stelle im Organismus implantiert und über die Verbindungsleitung mit dem Gehäuse verbunden.

Eine Einsatzmöglichkeit für die erfindungsgemäßen Dosiersysteme besteht vorzugsweise bei unter der Haut liegenden Knochenpartien von Mensch und Tier, die für eine Implantation des Dosiersystems bzw. seiner entsprechenden Teile ausreichend groß sind. Solche Knochenpartien sind beispielsweise an den Schädelknochen, ihren Höhlen oder den Beckenknochen des Menschen vorhanden. Ein derartiger Einsatz hat den Vorteil, daß das System bei entsprechendem Einbau von außen nicht erkennbar ist und auf diese Weise eine mögliche Stigmatisierung des Patienten erheblich reduziert wird. Bei bevorzugten Ausführungsformen der Erfindung ist die Form des Gehäuses dementsprechend für eine Implantation beim Menschen im Bereich der Schädelknochen, insbesondere in den Knochenhöhlenbereich hinter der Ohrmuschel ausgebildet. Dieser Knochenhöhlenbereich hinter der Ohrmuschel (Mastoid) stellt einen besonders guten Einsatzort des Dosiersystems für die Behandlung des Mittel- und Innenohrs dar, da er natürlicherweise mit dem Mittelohr und über die Membranen des runden und ovalen Fensters auch mit dem Innenohr in Verbindung steht.

Bei der Erfindung bildet das Gehäuse vorzugsweise einen ersten Abschnitt und einen innerhalb des Gehäuses unmittelbar an den ersten Abschnitt angrenzenden zweiten Abschnitt. Bei diesen Ausführungsformen ist die Pumpe vorzugsweise in dem den ersten Abschnitt bildenden Teil des Gehäuses vorgesehen. Insbesondere kann dann auch gleich das Betätigungselement für die Pumpe am ersten Abschnitt angeordnet sein. So kann der erste Abschnitt von einem im wesentlichen zylinderförmigen Teil des Gehäuses gebildet sein, in dem die Pumpe vertikal (senkrecht zur Oberseite des Gehäuses) angeordnet ist und in dem sich an der Oberseite des Zylinders das Betätigungselement und an der Unterseite des Zylinders die Austrittsöffnung der Pumpe befindet. In Weiterbildung ist die sich selbsttätig verschließende Öffnung am zweiten Abschnitt des Systems vorgesehen. Dadurch wird unter anderem erreicht, daß das Betätigen der Pumpe und das Wiederbefüllen des Medikamentenspeichers an verschiedenen Stellen des Systems erfolgen kann.

Eine Belüftung des Medikamentenspeichers könnte grundsätzlich über eine Belüftungsleitung oder passiv über eine semipermeable Membran, z.B. aus porösem PTFE oder Silikon, erfolgen. Wird ein (separater) elastischer Medikamentenspeicher (z.B. aus Silikon) verwendet, kann aber auf einen Belüftungsmechanismus verzichtet werden. Das durch die Pumpapplikation entnommene Medikamentenvolumen wird dabei durch Verformung der Speichergeometrie (Veränderung des Speichervolumens) ausgeglichen.

Das Betätigungselement, z.B. ein Betätigungsknopf, besteht nach der Erfindung vorzugsweise im wesentlichen aus einer Druckfläche, die mit der Pumpe, beispielsweise dem Druckkolben der Kolbenpumpe zusammenwirkt. Diese Druckfläche kann vorzugsweise eine im wesentlichen elastische Deckmembran aufweisen, über die bei transkutaner Betätigung der Finger auf die Pumpe einwirkt. Das Betätigungselement, insbesondere die Deckmembran, kann leicht erhaben ausgebildet sein, d.h. zumindest teilweise gegenüber ihrer Umgebung beispielsweise dem Gehäusedeckel erhöht sein. Dadurch ist die Betätigungsstelle für den Patienten leichter auffindbar und die Bedienbarkeit des Dosiersystems weiter verbessert. Der Durchmesser des Betätigungselements ist zweckmäßig an die Größe eines zur Betätigung benutzten Fingers angepaßt und beträgt vorzugsweise ca. 10 bis 20 mm. Vorzugsweise ist eine automatische Rückstellung des Betätigungselements, beispielsweise durch eine Federfunktion vorgesehen. Die Betätigungskraft ist vorzugsweise so groß eingestellt, daß ein unbeabsichtigtes Betätigen des Systems (z.B. durch Kopfbewegungen des Patienten im Schlaf) ausgeschlossen werden kann. Es ist aber andererseits vorteilhaft, wenn die Betätigungskraft nicht so groß ist, daß Gewebeschädigungen (Drucknekrosen) des implantatüberdeckenden Gewebes oder Implantatlagers auftreten können. Der Betätigungshub des Betätigungselementes liegt vorzugsweise im Bereich zwischen 1 und 2 mm. Die Betätigungskräfte können insbesondere 1 bis 5 N (Newton), vorzugsweise 1 bis 2 N, betragen.

Bei der erfindungsgemäß vorhandenen Öffnung, die sich selbsttätig verschließt, handelt es sich um ein sogenanntes "Port", durch das der Medikamentenspeicher beispielsweise entleert, gereinigt, desinfiziert, be- und entlüftet und wiederbefüllt werden kann. Ein solches Port ist vorzugsweise durch eine geeignete Membran wie beispielsweise ein sog. Einstich-Septum gebildet. In diesen Fällen wird der Medikamentenspeicher mit Hilfe von Spezialkanülen entleert oder befüllt. Durch entsprechende Wahl des Einstich-Septums kann der Vorgang sehr oft wiederholt werden. In Weiterbildung kann an der Öffnung ein Zentrierradius vorhanden sein, durch den die Membran etwas tiefergelegt ist als die Oberfläche des entsprechenden Gehäuses. Dadurch wird das Auffinden der Öffnung am implantierten Gehäuse und das Einstechen der Spezialkanüle erleichtert. Zur leichteren Erkennbarkeit des Ports kann auf der Oberfläche der Membran oder auf der über dem Port angeordneten Stelle der Haut ein entsprechendes Raster aufgebracht sein.

Die Größe des Medikamentenspeichers ist abhängig von der vorgesehenen Verwendung im wesentlichen frei wählbar. Volumina bis zu 10 cm³ sind, da ein getrennter (elastischer) Medikamtenspeicher vorgesehen ist, ohne weiteres realisierbar. Zweckmäßig sind, insbesondere bei einem Einsatz des Gesamtsystems im Bereich des Mastoids, Volumina zwischen ca. 1 und 5 cm³, insbesondere Volumina zwischen ca. 1 und 2,5 cm³, vorzugsweise zwischen ca. 1 und 2 cm³. Durch diese relativ großen Mengen bei gleichzeitig vergleichsweise geringen Abmessungen des Gesamtsystems muß der Patient vergleichsweise selten zur Wiederbefüllung des Medikamentenspeichers einbestellt werden. Dadurch können u.a. die personellen Behandlungskosten beträchtlich gesenkt werden.

Nach der Erfindung ist die Pumpe vorzugsweise zur diskontinuierlichen Dosierung von Mengen im Mikroliterbereich vorgesehen (Mikrodosiersystem). Auf diese Weise können geringe, fest einstellbare Mengen appliziert werden. Bevorzugte Dosierungen liegen zwischen 1 und 100 µl, innerhalb dieses Bereichs zwischen 5 und 10 µl und zwischen 10 und 20 µl pro Betätigung des Systems.

Bei der Erfindung wird eine Kolbenpumpe, nämlich eine sogenannte Tauchkolbenpumpe verwendet. Diese weist ein Saugventil für das Fluid, das bei Überdruck in der Pumpenkammer sperrt und bei Unterdruck öffnet, sowie ein als Schlauchventil ausgebildetes Druckventil für das Fluid, das bei Unterdruck in der Pumpenkammer sperrt und bei Überdruck öffnet, auf. Das Saugventil und das Druckventil arbeiten wechselseitig in Abhängigkeit vom hydrostatischen Druck in der Pumpenkammer.

Falls u.U. erforderlich, kann bei der Pumpe ein als Belüftungseinrichtung dienendes Belüftungsventil vorgesehen sein. Dieses Belüftungsventil öffnet bei ausreichend großem Unterdruck im Medikamentenspeicher selbsttätig und ermöglicht so den Druckausgleich.

Weiterhin kann das System einen akustischen Signalgeber aufweisen, der beispielsweise die Pumpenfunktion angibt. Auf diese Weise wird dem Patienten akustisch mitgeteilt, daß die Betätigung der Pumpe ausgelöst und die Dosierung des Medikaments erfolgt ist. In diesem Fall ist der Signalgeber vorzugsweise an der Pumpe selbst angebracht, beispielsweise mit dem Hub des Kolbens gekoppelt. Eine Signalgebung kann auch zur Anzeige des Füllstands des Medikamentenspeichers vorgesehen sein, so daß dem Patienten rechtzeitig angezeigt wird, wenn eine Wiederbefüllung des Speichers erforderlich wird. Der Füllgrad des Medikamentenspeichers kann auch mit Hilfe von Ultraschall visualisiert werden.

Die Dosierleitung zwischen Austrittsöffnung der Pumpe und Applikationsort des Medikaments weist vorzugsweise mindestens an einem ihrer Enden ein Verschlußventil auf. Dieses Verschlußventil ist vorzugsweise an dem dem Applikationsort zugeordneten Ende der Leitung vorgesehen. Auf diese Weise wird ein Einwachsen von Gewebe und damit ein Zusetzen der Leitung verhindert. Zum Erreichen des gleichen Zwecks kann zusätzlich oder alternativ der Innendurchmesser der Dosierleitung möglichst klein gewählt werden. Dadurch und durch weitere Maßnahmen kann die Dosierleitung in zweckmäßiger Weise zur Applikation eines Therapeutikums in das Mittelohr ausgebildet sein.

Wie bereits geschildert, ist das erfindungsgemäße Dosiersystem zur Bereitstellung eines Therapeutikums vorgesehen. Dabei ist diese Verwendung insbesondere auf die Bereitstellung eines Therapeutikums zur Lokaltherapie des Mittel- und Innenohrs gerichtet. Vorzugsweise wird dabei das Innenohr mit Hilfe einer Applikation eines Medikaments, Wirkstoffs oder dgl. in das Mittelohr therapiert. Eine entsprechende Verwendung liegt insbesondere bei der Behandlung des Tinnitus, von Morbus Ménière, von chronischen Mittelohrentzündungen und bei der Schmerztherapie im Schädelbereich. Dabei ist vor allem das Mastoid zur Aufnahme des Dosiersystems oder eines Teiles davon (Gehäuse mit Pumpe und Port) geeignet. Vorzugsweise erfolgt die Fixierung im Knochenbereich hinter der Ohrmuschel (Mastoid) des Menschen. Zur Fixierung im Implantatlager können geeignete Schrauben, beispielsweise Titanschrauben oder Spezialzemente wie beispielsweise Glasionomerzement verwendet werden. Eine Fixierung mit chirurgischem Nahtmaterial und/oder Einwachsen von Knochenzellen in die Oberflächenrauhigkeit des Gehäusematerials (Titan) ist ebenfalls möglich.

Zur Implantation des von der Erfindung zur Verfügung gestellten Dosiersystems wird in diesem Fall der Knochenhöhlenbereich hinter der Ohrmuschel in entsprechender Form weiter ausgebohrt, wobei die als bevorzugt beschriebene Form des Gehäuses dem Aufbau des Mastoids in günstiger Weise entspricht. Anschließend wird eine Verbindung in die Paukenhöhle (aditus ad antrum oder durch die hintere Gehörgangswand) geschaffen oder genutzt, durch die eine Dosierleitung in die Paukenhöhle eingebracht wird. Anschließend wird das Dosiersystem/Gehäuse in der geschaffenen Höhle fixiert. Zur Vereinfachung der Implantierung kann das erfindungsgemäße Dosiersystem in bestimmten standardisierten Größen zur Verfügung gestellt werden, die in verschiedenen Größen der entsprechenden Knochenpartie hinter dem Ohr angepaßt sind. Der getrennte Medikamentenspeicher mit oder ohne Fort kann an anderer Stelle, beispielsweise im Halsbereich, Achselbereich oder am Hinterkopf vorgesehen sein. Die in den Durchbruch eingebrachte Dosierleitung kann bei der Anwendung zur Mittel- und Innenohrtherapie an nahezu beliebigen Orten im Mittelohr, so z.B. in der Nähe des sogenannten runden Fensters fixiert werden, beispielsweise mit Spezialzementen oder einem Titanclip. Der Wirkstoff kann dabei durch die Rundfenstermembran in die Lymphe des Innenohrs eindiffundieren. Es ist auch möglich, eine Dosierleitung direkt mit der Flüssigkeit des Innenohres oder des Gleichgewichtsorgans in Verbindung zu bringen.

Die beschriebenen Merkmale und weitere Merkmale der Erfindung ergeben sich aus der nachfolgenden Beschreibung von bevorzugten Ausführungsformen in Verbindung mit den Unteransprüchen und den Zeichnungen.

In den Zeichnungen zeigen:
- Fig. 1: die schematische Gesamtansicht eines Dosiersystems nach der Erfindung,
- Fig. 2: die schematische Gesamtansicht eines zweiten Dosiersystems nach der Erfindung,
- Fig. 3: die Schnittansicht eines Dosiersystems, die wesentliche Teile seines Aufbaus im implantierten Zustand zeigt.

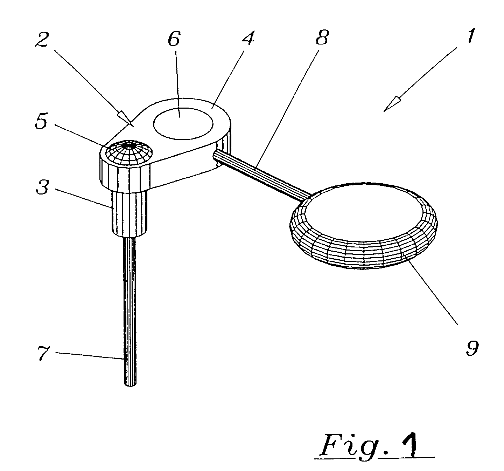

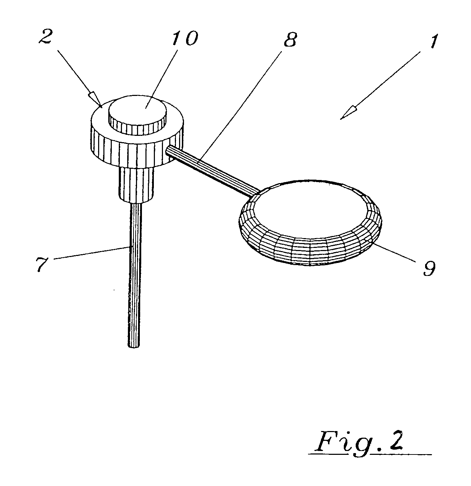

In den Fig. 1 und 2 sind zwei Ausführungsformen der Erfindung mit einem separaten Medikamentenspeicher, vorzugsweise aus elastischem Material dargestellt.

Das Dosiersystem 1 gemäß Fig. 1 besteht aus einem Gehäuse 2, das aus einem starren biokompatiblen Material besteht. In einem zylinderförmigen Abschnitt 3 des Gehäuses 2 ist eine in Fig. 1 nicht näher dargestellte Pumpe untergebracht, die mit Hilfe eines Betätigungsknopfes 5 betätigt werden kann. An einem flacheren weiteren Abschnitt 4 des Gehäuses 2 ist ein Port (Septum) 6 vorgesehen. Eine Dosierleitung 7 führt von einer in Fig. 1 nicht dargestellten Austrittsöffnung am Gehäuseabschnitt 3 zum Applikationsort. Desweiteren weist das Dosiersystem 1 gemäß Fig. 1 einen separaten Medikamentenspeicher 9 aus einem elastischen bio-kompatiblen Material, insbesondere Kunststoffmaterial, auf. Dieser Medikamentenspeicher 9 ist mit dem Gehäuse 2, im dargestellten Fall dem flacheren Gehäuseabschnitt 4, über eine Verbindungsleitung 8 verbunden.

Das in Fig. 1 dargestellte Gehäuse 2 kann beispielsweise eine Höhe von 1 bis 2 cm (in vertikaler Richtung am ersten Abschnitt 3 entsprechend der Lage der eingebauten Pumpe) und eine Gesamtlänge von 2 bis 4 cm (an der Oberseite des Gehäuses) besitzen.

Das Dosiersystem 1 gemäß Fig. 2 besteht aus einem ebenfalls vorzugsweise starren Gehäuse 2, in dessen Innerem sich eine nicht näher dargestellte Pumpe befindet. An der Oberseite des Gehäuses 2 ist ein Betätigungselement 10 vorgesehen, das nicht nur zum Auslösen der Pumpenfunktion, sondern gleichzeitig auch als Port (Septum) vorgesehen ist. Dieses Betätigungselement 10 ist gegenüber der Oberseite des Gehäuses 2 erhaben ausgebildet, damit es sowohl beim Auslösen der Pumpenfunktion als auch beispielsweise beim Wiederbefüllen leicht lokalisierbar ist. Eine Dosierleitung 7 führt vom Gehäuse 2 zum Applikationsort, und der separate elastische Medikamentenspeicher 9 ist über eine Verbindungsleitung 8 mit dem Gehäuse 2 verbunden.

Bei den Ausführungen nach Fig. 1 und Fig. 2 ist insbesondere das Gehäuse 2 zur Implantation in das Mastoid des Menschen vorgesehen. Die Dosierleitung/Dosierkanüle 7 wird dabei von der Austrittsöffnung am Gehäuse 2 ins Mittelohr geführt und damit eine gezielte Therapie des Mittel- oder Innenohrs ermöglicht. Durch die elastische Ausbildung des Medikamentenspeichers 9 ist keine besondere Belüftungseinrichtung für das Mikrodosiersystem nach der Erfindung erforderlich. Wie bereits beschrieben, kann der elastische Medikamentenspeicher (Tank) beispielsweise im Bereich des Hinterhauptes, des Halses oder der Achselhöhle unter der Haut implantiert werden.

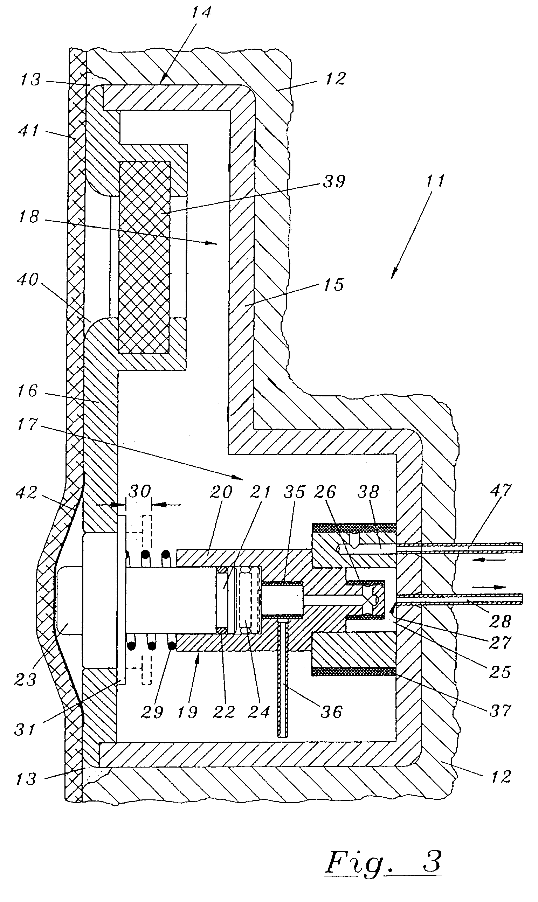

Aus Fig. 3 ist der Aufbau und die Funktionsweise des erfindungsgemäßen Dosiersystems, insbesondere der Tauchkolbenpumpe und des Schlauchventils zu erkennen. Hierbei wird insbesondere auf die untere Hälfte der Fig. 3 Bezug genommen.

Das Dosiersystem 11 gemäß Fig. 3 ist in einer entsprechenden Ausnehmung des Knochens 12, in diesem Fall des Mastoids hinter der Ohrmuschel mit Hilfe eines Spezialzements 13 fixiert. Der erforderliche Stufenbohrkonus im Mastoid wird mit Hilfe einer speziellen mikrochirurgischen Bohrtechnik in den Knochen gefräst.

Das Gehäuse 14 des Dosiersystems 11 ist aus einem Gehäusebasisteil 15 und einem Gehäusedeckel 16 aufgebaut. Durch die gezeigte Form des Gehäusebasisteils 15 und des Gehäusedeckels 16 wird vom Gehäuse 14 ein erster Abschnitt 17 und ein zweiter Abschnitt 18 gebildet. Innerhalb des ersten Abschnittes 17 befindet sich die Pumpe, die im dargestellten Fall, aber nicht notwendigerweise, relativ zur Anordnung des Gehäuses 14 in nicht implantiertem Zustand in vertikaler Richtung eingebaut ist.

Gemäß Fig. 3 ist die Pumpe als Tauchkolbenpumpe 19 ausgebildet. Diese besteht aus dem Pumpengehäuse 20, in dem der Kolben 21 geführt und mit Hilfe des Dichtelements 22 abgedichtet ist. Durch axiale Verschiebung des Kolbens 21 bei Betätigung des mit dem Kolben zusammenwirkenden Betätigungsknopfs 23 wird das sich in der Pumpenkammer 24 befindende Fluid in die Auslaßkammer 25 überführt. Dabei wird das bei Unterdruck in der Pumpenkammer 24 sperrende Schlauchventil 26 zur Einleitung des Fluids in die Auslaßkammer 25 durch den entstandenen Überdruck geöffnet. Auf diese Weise kann das Fluid durch die mit der Austrittsöffnung 27 der Pumpe 19 verbundenen Dosierleitung 28 zum Applikationsort strömen. Nach Betätigung des Betätigungsknopfes 23 wird der Kolben 21 durch die Rückstellfeder 29 wieder in den Ausgangszustand (nicht ausgelösten Zustand) gebracht. Der Kolbenhub ist in Fig. 3 durch die Bezugsziffer 30 gekennzeichnet. Unterhalb des Gehäusedeckels 16 ist ein Anschlag 31 zur Begrenzung der Rückstellbewegung des Kolbens 21 vorgesehen.

Als Saugventil für das Fluid ist an der Pumpe 19 ein Schlauchventil 35 vorgesehen. Dieses Schlauchventil 35 sperrt bei Überdruck in der Pumpenkammer 24. Bei Unterdruck wird dieses Ventil geöffnet und das Fluid über die Saugleitung 36 in die Pumpenkammer 24 geleitet.

Wie bereits ausgeführt, ist bei der Erfindung üblicherweise keine Belüftung erforderlich, da ein elastischer separater Medikamentenspeicher verwendet werden kann. Gemäß Fig. 3 kann aber auch ein als Unterdruckventil für die Belüftung dienendes weiteres Schlauchventil 37 an der Pumpe 19 vorgesehen sein, das unabhängig von der Kolbenbewegung oder den Druckverhältnissen in der Pumpenkammer 24 öffnet oder schließt. Bei ausreichend großem Unterdruck im Medikamentenspeicher öffnet das Schlauchventil 37 selbsttätig und ermöglicht so den Druckausgleich. Die entsprechende Gaszufuhr für die Belüftung erfolgt über einen Ansaugkanal 38, der über eine Belüftungsleitung 47 beispielsweise mit der Mittelohrhöhle verbunden sein kann. Im zweiten Abschnitt 18 des Gehäuses 14 ist ein Port mit Einstich-Septum 39 vorgesehen. Durch dieses Einstich-Septum 39 kann der Medikamentenspeicher entleert, gereinigt, desinfiziert, be- und entlüftet oder befüllt werden. Um das Einführen einer Kanüle zu erleichtern, ist am Umfang des Einstich-Septums 39 ein Zentrierradius 40 vorgesehen.

Schließlich ist in Fig. 3 noch die elastische Kopfhaut 41 dargestellt, die nach dem Implantieren des Dosiersystems 11 wieder an ihren ursprünglichen Ort über der Stelle des Eingriffs gelegt wird. Zur Schonung des Gewebes ist an dem Bereich der Kopfhaut 41, an der sich der Betätigungsknopf 23 für die Pumpe 19 befindet, unterhalb der Kopfhaut 41 eine elastische Membran 42 vorgesehen, die relativ zum Gehäusedeckel 16 höher gelegt ist und an ihrem Randbereich mit dem Gehäusedeckel 16 dicht verbunden ist.

## Patentansprüche

1. Implantierbares Dosiersystem für in Form von insbesondere gelösten oder suspendierten Fluiden zu applizierende Medikamente, Wirkstoffe oder dgl., das
- einen separaten Medikamentenspeicher (9)
- ein Gehäuse (2, 14) mit
einer innerhalb des Gehäuses angeordneten Pumpe (19), die mit Hilfe eines am Gehäuse befindlichen Betätigungselements (5; 10; 23) transkutan betätigbar ist,
einer Austrittsöffnung (27) für das zu applizierende Medikament,
einem am Auslaß der Pumpe angeordneten Ventil,
einer sich selbsttätig verschließenden Öffnung (6; 39), insbesondere zur Wiederbefüllung des Medikamentenspeichers,
- eine Verbindungsleitung (8) zwischen Gehäuse (2) und dem Medikamentenspeicher (9),
- und ggf. eine von der Austrittsöffnung (27) zum Applikationsort führende Dosierleitung (7; 28) aufweist,
dadurch gekennzeichnet, daß es sich bei der Pumpe um eine Tauchkolbenpumpe (19) handelt, das Ventil als fluiddruckabhängig öffnendes Schlauchventil (26) ausgebildet ist und die Austrittsöffnung (27) durch Betätigung des Betätigungselements (5; 10; 23) der Pumpe (19) über das Schlauchventil (26) reversibel mit Fluid beaufschlagbar ist.

2. Dosiersystem nach Anspruch 1, gekennzeichnet durch ein formstabiles, im wesentlichen starres Gehäuse (2, 14) aus einem Material ausreichender Festigkeit.

3. Dosiersystem nach Anspruch 1 oder 2, dadurch gekennzeichnet, daß das Gehäuse (2, 14) aus einem biokompatiblen Kunststoff, insbesondere Polyurethan und/oder einem biokompatiblen Metall, insbesondere Titan aufgebaut ist.

4. Dosiersystem nach einem der vorhergehenden Ansprüche, dadurch gekennzeichnet, daß das Gehäuse (14) aus einem Gehäusebasisteil (15) und einem Gehäusedeckel (16) aufgebaut ist.

5. Dosiersystem nach einem der vorhergehenden Ansprüche, dadurch gekennzeichnet, daß die Form des Gehäuses (2, 14), insbesondere die Form des Gehäusebasisteils, für eine Implantation des Dosiersystems beim Menschen in den Knochenbereich hinter der Ohrmuschel ausgebildet ist.

6. Dosiersystem nach einem der vorhergehenden Ansprüche, dadurch gekennzeichnet, daß der Medikamentenspeicher (9) aus einem elastischen biokompatiblen Kunststoffmaterial besteht.

7. Dosiersystem nach einem der vorhergehenden Ansprüche, dadurch gekennzeichnet, daß der Medikamentenspeicher (9) zur Implantation durch Fixieren an der Muskelfaszie, insbesondere im Bereich der Achselhöhle, des Halses oder des Hinterhauptes, vorgesehen ist.

8. Dosiersystem nach einem der vorhergehenden Ansprüche, dadurch gekennzeichnet, daß das Betätigungselement (10) gegenüber dem Gehäuse (2) erhaben ausgebildet ist.

9. Dosiersystem nach einem der vorhergehenden Ansprüche, dadurch gekennzeichnet, daß das Betätigungselement (23) zur transkutanen Betätigung der Pumpe (19) von einer Druckfläche gebildet ist, die insbesondere eine im wesentlichen elastische Membran aufweist.

10. Dosiersystem nach einem der vorhergehenden Ansprüche, dadurch gekennzeichnet, daß die sich selbsttätig verschließende Öffnung (6) durch ein Einstich-Septum (39) gebildet ist.

11. Dosiersystem nach einem der vorhergehenden Ansprüche, dadurch gekennzeichnet, daß das Betätigungselement und die sich selbsttätig verschließende Öffnung als bauliche Einheit ausgebildet sind, wobei insbesondere die zur transkutanen Betätigung vorgesehene Druckfläche von einem Einstichseptum gebildet ist.

12. Dosiersystem nach einem der vorhergehenden Ansprüche, dadurch gekennzeichnet, daß der Medikamentenspeicher (9) ein Volumen von ca. 1 bis 5 cm³, insbesondere ca. 1 bis 2,5 cm³, besitzt.

13. Dosiersystem nach einem der vorhergehenden Ansprüche, dadurch gekennzeichnet, daß die Pumpe zur diskontinuierlichen Applikation von Volumina zwischen 1 und 100 µl, insbesondere 10 bis 20 µl, ausgebildet ist.

14. Dosiersystem nach einem der vorhergehenden Ansprüche, dadurch gekennzeichnet, daß die Dosierleitung vorzugsweise an mindestens einem ihrer beiden Enden, insbesondere an dem dem Applikationsort zugeordneten Ende, ein Verschlußventil aufweist.

## Claims

1. Implantable dosaging system for medications, active substances or the like more particularly to be administered in the form of dissolved or suspended fluids, which has
- a separate medication reservoir (9),
- a housing (2, 14) having
a pump (19) positioned within the housing and which is transcutaneously operable with the aid of an actuator (5, 10, 23) located on the housing,
a discharge opening (27) for the medication to be administered,
a valve located at the pump outlet,
a self-sealing port (6, 39), particularly for refilling the medication reservoir,
- a connecting line (8) between the housing (2) and the medication reservoir (9),
- and optionally a dosaging line (7, 28) leading from the discharge opening (27) to the administration location,
characterized in that the pump is a plunger pump (19), the valve is constructed as a fluid Pressure-dependent opening pipe compression valve (26) and the discharge opening (27) can be reversibly supplied with fluid by operating the actuator (5, 10, 23) of the pump (9) by means of the pipe compression valve (26).

2. Dosaging system according to claim 1, characterized by a dimensionally stable, substantially rigid housing (2, 14) made from a material having an adequate strength.

3. Dosaging system according to claim 1 or 2, characterized in that the housing (2, 14) is formed from a biocompatible plastic, particularly polyurethane and/or a biocompatible metal, Particularly titanium.

4. Dosaging system according to one of the preceding claims, characterized in that the housing (14) is formed from a housing base part (15) and a housing lid (16).

5. Dosaging system according to one of the preceding claims, characterized in that the shape of the housing (2, 14), particularly the shape of the housing base part is suitable for a implantation of the dosaging system in humans, in the bone region behind the auricle.

6. Dosaging system according to one of the preceding claims, characterized in that the medication reservoir (9) is made from an elastic, biocompatible plastics material.

7. Dosaging system according to one of the preceding claims, characterized in that the medication reservoir (9) is intended for implantation by fixing to the muscular fascia, particularly in the vicinity of the axilla, neck or occiput.

8. Dosaging system according to one of the preceding claims, characterized in that the actuator (10) is raised compared with the housing (2).

9. Dosaging system according to one of the preceding claims, characterized in that the actuator (23) for the transcutaneous operation of the pump (19) is formed by a pressure surface, which in particular has a substantially elastic membrane.

10. Dosaging system according to one of the preceding claims, characterized in that the self-sealing port (39) is formed by a puncture septum (39).

11. Dosaging system according to one of the preceding claims, characterized in that the actuator and self-sealing port are constructed as a unit and in particular the pressure surface provided for transcutaneous operation is formed by a puncture septum.

12. Dosaging system according to one of the preceding claims, characterized in that the medication reservoir (9) has a volume of approximately 1 to 5 cm³, particularly approximately 1 to 2.5 cm³.

13. Dosaging system according to one of the preceding claims, characterized in that the pump is constructed for the discontinuous administration of volumes between 1 and 100 µl, particularly 10 to 20 µl.

14. Dosaging system according to one of the preceding claims, characterized in that the dosaging line has preferably at at least one of its two ends, particularly the end associated with the administration point, a closure valve.

## Revendications

1. Système de dosage pouvant être implanté pour médicaments, substances actives ou similaires, qui sont appliquées sous la forme de fluides en particulier dissous ou en suspension, qui présente
- un réservoir séparé à médicament (9),
- un boîtier (2; 14) avec
une pompe (19) disposée à l'intérieur du boîtier, qui peut être actionnée par voie trenscutanée à l'aide d'un élément d'actionnement (5; 10; 23) se trouvant sur le boîtier,
un orifice de sortie (27) pour le médicament à appliquer,
une vanne disposée à la sortie de la pompe,
un orifice (6; 39) se refermant automatiquement, en particulier pour le nouveau remplissage du réservoir à médicament,
- une tuyauterie de liaison (8) entre le boîtier (2) et le réservoir à médicament (9),
- et le cas échéant une tuyauterie de dosage (7; 28) conduisant de l'orifice de sortie (27) vers le lieu d'application,
caractérisé en ce que la pompe est une pompe à piston plongeur (19), la vanne est construite comme vanne de chambre à air (26) s'ouvrant en fonction de la pression du fluide et l'orifice de sortie (27) peut être mis de manière réversible en contact avec le fluide par l'intermédiaire de la vanne de chambre à air (26) par actionnement de l'élément d'actionnement (5; 10; 23) de la pompe (19).

2. Système de dosage suivant la revendication 1, caractérisé par un boitier (2; 14) indéformable, essentiellement rigide, fabriqué dans un matériau de résistance suffisante.

3. Système de dosage suivant la revendication 1 ou 2, caractérisé en ce que le boîtier (2; 14) est construit dans une matière plastique biocompatible, en particulier en polyuréthanne, et/on un métal biocompatible, en particulier en titane.

4. Système de dosage suivant l'une des revendications précédentes, caractérisé en ce que le boîtier (14) est constitué d'une pièce de base de boîtier (15) et d'un couvercle de boîtier (16).

5. Système de dosage suivant l'une des revendications précédentes, caractérisé en ce que la forme du boîtier (2; 14), en particulier la forme de la pièce de base de boîtier, est adaptée à une implantation du système de dosage chez l'être humain dans la zone osseuse située derrière le pavillon de l'oreille.

6. Système de dosage suivant l'une des revendications précédentes, caractérisé en ce que le réservoir à médicament (9) est constitué d'une matière plastique élastique biocompatible.

7. Système de dosage suivant l'une des revendications précédentes, caractérisé en ce que le réservoir à médicament (9) est prévu pour implantation par fixation aux fibres musculaires, en particulier dans la zone de l'aisselle, du cou ou de la nuque.

8. Système de dosage suivant l'une des revendications précédentes, caractérisé en ce que l'élément d'actionnement (10) est formé légèrement en saillie par rapport au boîtier (2).

9. Système de dosage suivant l'une des revendications précédentes, caractérisé en ce que l'élément d'actionnement (23) pour actionnement transcutané de la pompe (19) est formé par une surface de pression, qui présente en particulier une membrane essentiellement élastique.

10. Système de dosage suivant l'une des revendications précédentes, caractérisé en ce que l'élément d'actionnement et l'orifice se refermant automatiquement (6) sont constitués par un septum d'injection (39).

11. Système de dosage suivant l'une des revendications précédentes, caractérisé en ce que l'élément d'actionnement et l'orifice se refermant automatiquement sont construits sous la forme d'une unité constructive, la surface de pression prévue pour actionnement transcutané étant en particulier constituée par un septum d'injection.

12. Système de dosage suivant l'une des revendications précédentes, caractérisé en ce que le réservoir à médicament (9) possède un volume d'environ 1 à 5 cm³, en particulier d'environ 1 à 2,5 cm³.

13. Système de dosage suivant l'une des revendications précédentes, caractérisé en ce que la pompe est conçue pour l'application discontinue de volumes compris entre 1 et 100 µl, en particulier entre 10 et 20 µl.

14. Système de dosage suivant l'une des revendications précédentes, caractérisé en ce que la tuyauterie de dosage présente une vanne de fermeture de préférence à au moins une de ses extrémités, en particulier à l'extrémité située du côté du point d'application.
